# EUROPEAN PATENT SPECIFICATION

(11) **EP 2 880 978 B1**
(45) Date of publication and mention of the grant of the patent: **04.10.2017**
(21) Application number: 13461564.0
(22) Date of filing: 09.12.2013
(51) Int. Cl.: C07C 59/70, C07C 211/63, C07C 215/12, C07C 217/28, C07C 219/06, C07C 219/08, A01N 39/04

(54) **Herbicidal quaternary ammonium salts of (4-chloro-2-methylphenoxy)acetic acid**
Herbizid-wirkende quaternäre Ammoniumsalze von (4-Chloro-2-methylphenoxy)essigsäure
Herbicide à base de sels quaternaires d'ammonium de l'acide (4-chloro-2-méthylphénoxy)acétique

(43) Date of publication of application: 10.06.2015
(73) Proprietor: Przedsiebiorstwo Produkcyjno-Consultingowe ADOB sp. z o.o. sp. k., 61-070 Poznan (PL)
(72) Inventor: Nawrocki, Adam, 61-306 Poznan (PL); Pernak, Juliusz, 60-573 Poznan (PL); Praczyk, Tadeusz, 62-030 Lubon (PL); Olszewski, Radoslaw, 61-038 Poznan (PL); Niemczak, Michal, 88-100 Inowroclaw (PL)
(74) Representative: Sitkowska, Jadwiga

(56) References cited:
- WO-A1-2008/140338
- GB-A- 1 078 804
- JULIUSZ PERNAK ET AL: "Ionic liquids with herbicidal anions", TETRAHEDRON, ELSEVIER SCIENCE PUBLISHERS, AMSTERDAM, NL, vol. 67, no. 26, 5 May 2011 (2011-05-05), pages 4838-4844, XP028227648, ISSN: 0040-4020, DOI: 10.1016/J.TET.2011.05.016 [retrieved on 2011-05-12]

## Description

### Field of the invention

The present invention relates to the field of herbicidal agents. In particular, it relates to novel quaternary ammonium salts of known herbicide (4-chloro-2-methylphenoxy)acetic acid. Novel ammonium salts are ionic liquids and are useful as herbicides in plant protection.

### Background art

(4-Chloro-2-methylphenoxy)acetic acid (common name MCPA) is a popular herbicide that belongs to the group of phenoxyacids. MCPA is a selective herbicide having systemic activity similar to the activity of auxins, natural growth regulators. MCPA is a low cost herbicide commonly used in agriculture for protection of a large variety of plants, including cereals, potatoes and flax, in fruit-farming, as well as for protection of grassland. It penetrates plant leafs easily, then distributes quickly and accumulates mainly in growth zones. Although MCPA is very effective against sensitive weeds, to enhance its herbicidal activity it is often used in mixtures with other herbicidally active compounds, such as dichlorprop (2-(2,4-dichlorophenoxy)-propionic acid), mecoprop (2-(4-chloro-2-methylphenoxy)propionic acid), dicamba (3,6-dichloro-2-methoxybenzoic acid), and fluroxypyr ([(4-amino-3,5-dichloro-6-fluoro-2-pyridinyl)oxy]acetic acid]).

One of disadvantages of MCPA is that due to the presence of carboxyl group in its molecule it can form under suitable pH conditions stable complexes with the species present in the soil, including metal ions, such as Hg, Co, Pb and Cd. This results in the change of properties of this active substance. Complexes of MCPA with metal ions are insoluble and absorbed by the soil and their biodegradation is slower. Thus, the possibility of formation of complexes with metal is dangerous for the environment due to accumulation of these compounds in the plants and the soil.

The solution of this problem proposed in the prior art were salts of the MCPA anion formed with certain quaternary ammonium cations. Such salts having a melting point below 100°C belong to the group of chemical compounds called ionic liquids.

Ionic liquids are built of an organic or inorganic anion and an organic cation. A characteristic feature which allows to classify a compound to the group of ionic liquids is its melting point, which by definition can not exceed 100°C.

Ionic liquids comprising MCPA anion are disclosed in WO2008/140338. WO2008/140338 discloses, inter alia, ionic pairs of MCPA anion with methyltri(R)-, dimethyldi(R)-, and trimethyl(R)ammonium cations, where R can be a linear alkyl group containing 1 to 18 carbon atoms or a mixture of linear alkyl chains containing 1 to 20 carbon atoms (common names cocoalkyl or hydrogenated tallowalkyl), or polyoxyethylene group. Such ionic pairs are disclosed only generally, without exemplification of any specific compound having such structure. Ionic liquids comprising MCPA anion are disclosed also in J. Pernak et al., Tetrahedron 67(2011), 4838-4844. Among many others, disclosed are ionic pairs of MCPA anion with quaternary di(hydrogenated tallow alkyl)dimetyl and di(C₁₀H₂₁ alkyl)dimethyl ammonium cations, i.e. di(hydrogenated tallow)dimethylammonium (4-chloro-2-methylphenoxy)acetate and didecyldimethylammonium (4-chloro-2-methyl-phenoxy)acetate, respectively.

Prior art ionic pairs have herbicidal activity due to the presence of MCPA anion. Several further advantages of MCPA anion containing ionic liquids are disclosed generally, i.e. reduced volatility and drift during and after application, thermal and chemical resistance, lack of reaction with metal ions due to the absence of free carboxylate group and resulting inhibition of the transport of a metal to the soil, limited tendency to deposit in natural environment and therefore more environmental friendliness comparing to the parent herbicide - MCPA, as well as additional surfactant properties.

Despite of described advantages of known quaternary ammonium ionic liquids containing MCPA anion, a need still exists of herbicidal compounds having high herbicidal activity.

Furthermore, a need still exits of novel herbicidal compounds having higher herbicidal activity than known compounds without loss of other advantageous properties of known compounds. This would allow to lower dosage of a herbicide and thus protect the environment.

A need also exists of novel herbicidal compounds that are more prone to biodegradation.

There is also a need of novel herbicides having consistence that makes their application easier.

There is also a need of novel herbicides that are less prone to acquiring resistance by controlled weeds.

These objects have been realized by novel ionic liquids - salts of the invention comprising MCPA anion.

### Description of the invention

The present invention relates to a salt compound represented by the following Formula (1) 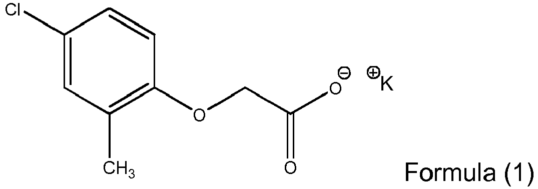 wherein K represents a quaternary ammonium cation of the formula of the formula selected from the group consisting of Formula (2), Formula (3), Formula (4), and Formula (5), 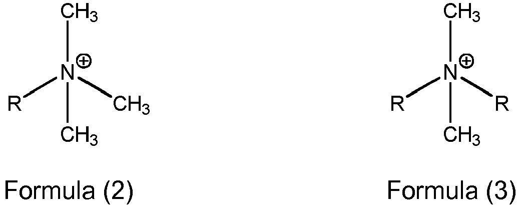 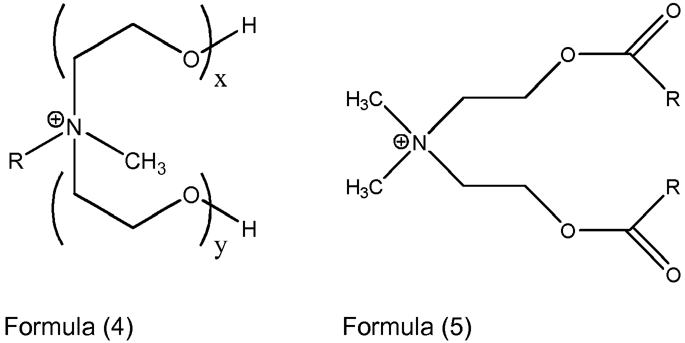 wherein R represents an alkyl selected from the group consisting of tallow, soya and oleyl alkyl, and
x and y in Formula (4) are integers from the range 1 to 15, inclusive.

One embodiment of the invention is the salt of the Formula (1) as defined above, wherein R represents tallow alkyl.

Another embodiment of the invention is the salt of the Formula (1) as defined above, wherein R represents soya alkyl.

Another embodiment of the invention is the salt of the Formula (1) as defined above, wherein R represents oleyl alkyl.

One group of the salts of the invention are the salts wherein K in Formula (1) is selected from the group consisting of Formula (2) and Formula (3).

Another group of the salts of the invention are the salts wherein K in Formula (1) is selected from the group consisting of Formula (4) and Formula (5).

Another embodiment of the invention are the salts of Formula (1), wherein K in Formula (1) is represented by Formula (2).

Another embodiment of the invention are the salts of Formula (1), wherein K in Formula (1) is represented by Formula (3).

Yet another embodiment of the invention are the salts of Formula (1), wherein K in Formula (1) is represented by Formula (4).

Yet another embodiment of the invention are the salts of Formula (1), wherein K in Formula (1) is represented by Formula (5).

Representative specific compounds of the invention are selected from the group consisting of:
- trimethylsoyaammonium (4-chloro-2-methylphenoxy)acetate,
- dimethylditallowammonium (4-chloro-2-methylphenoxy)acetate
- bis(2-hydroxyethyl)methyloleylammonium (4-chloro-2-methylphenoxy)acetate, and
- dimethyldi(tallowoyloxyethyl)ammonium (4-chloro-2-methylphenoxy)acetate.

The salts of the invention are ionic liquids having herbicidal activity and are useful as plant protection agents for combating and controlling undesirable vegetation, such as weeds.

Accordingly, the present invention relates to a herbicidal composition, which comprises a herbicidally effective amount of the salt of Formula (1) as defined above, and a carrier.

The present invention relates also to a method of combating undesirable plant vegetation, which comprises treating said undesirable plant vegetation or a locus thereof with a herbicidally effective amount of the salt of Formula (1) as defined above or the composition comprising said salt.

In the present description and claims the terms tallow alkyl, soya alkyl and oleyl alkyl shall have, in accordance with their common understanding in the art, the following meanings.

The term "tallow alkyl" or "tallow" relates to a mixture of saturated and unsaturated alkyl groups composed of alkyl groups with 12 carbon atoms, 14 carbon atoms, 16 carbon atoms, and 18 carbon atoms, wherein the percentage of groups with double bonds is 40 to 55%, the percentage of alkyl groups with 16 carbon atoms is 25 to 35%, and the percentage of alkyl groups with 18 carbon atoms is 60 to 70%.

The term "soya alkyl" or "soya" relates to a mixture of saturated and unsaturated alkyl groups composed of alkyl groups with 14 carbon atoms, 16 carbon atoms, and 18 carbon atoms, wherein the percentage of groups with double bonds is 55 to 70%.

The term "oleyl alkyl" or "oleyl" relates to a mixture of saturated and unsaturated alkyl groups composed of alkyl groups having 12 carbon atoms, 14 carbon atoms, 16 carbon atoms, and 18 carbon atoms, wherein the percentage of groups with double bonds is 75 to 82%, and the percentage of alkyl groups with 18 carbon atoms is 75 to 85%.

As it can be seen, novel salt of the invention has quaternary ammonium cation with at least one long alkyl substituent containing high fraction of alkyls with unsaturated double bonds in a chain, unlike prior art ionic liquids of MCPA anion wherein long alkyl substituent in the cation contains solely saturated alkyl groups.

Novel salts of the invention have herbicidal activity due to the presence of (4-chloro-2-methylphenoxy)acetate anion, and have advantageous properties similar to those disclosed for the prior art compounds, namely:
- due to low melting point below 100°C are ionic liquids,
- have high thermal and chemical stability,
- are non-volatile - their vapor pressure at ambient temperature is in practice non-measurable,
- can be hydrophobic or hydrophilic, depending on the type of substituents in the quaternary ammonium cation,
- due to the presence of a cation with at least one long alkyl substituent are cationic surfactants,
- do not form complexes with heavy metals, which results in inhibition of the transport of a heavy metal to the protected plant,
- due to the presence of large cation possess also bactericidal and fungicidal properties, and can be considered multifunctional compounds.

However, in addition to the above advantages, the salts of the invention unexpectedly and surprisingly may have higher herbicidal activity than prior art compounds with MCPA anion and a quaternary ammonium cation described above.

Also surprisingly and unexpectedly, the salts of the invention due to the presence of alkyl groups with high fraction of unsaturated bonds in the quaternary ammonium cation are characterized by higher biodegradation rate than prior art compounds with a cation having only saturated alkyl chains.

In addition to the advantageous properties discussed above:
- the presence of unsaturated bonds enhances the oxidability potential of the compounds, diminishing their accumulation in the environment and making them more environment-friendly,
- the cost of reagents with mixed saturated and unsaturated alkyl groups used for the preparation of the salts of the invention is lower comparing to reagents having only saturated substituents, while high activity of the ionic liquid is maintained,
- the presence of both saturated and unsaturated substituents in the mixture influences the consistence of the resulting ionic liquids in a manner which makes their adaptation to the equipment or usage form requirements easier,
- the use of a herbicide having mixture of saturated and unsaturated substituents significantly lowers the risk of acquiring resistance by combated weeds, as it happens with conventional agents.

The salts of the invention of the Formula (1) as defined above, wherein K represents Formula (2), Formula (3), Formula (4) or Formula (5), can be obtained from a quaternary ammonium halide of the Formula (6), Formula (7), Formula (8) or Formula (9), respectively, 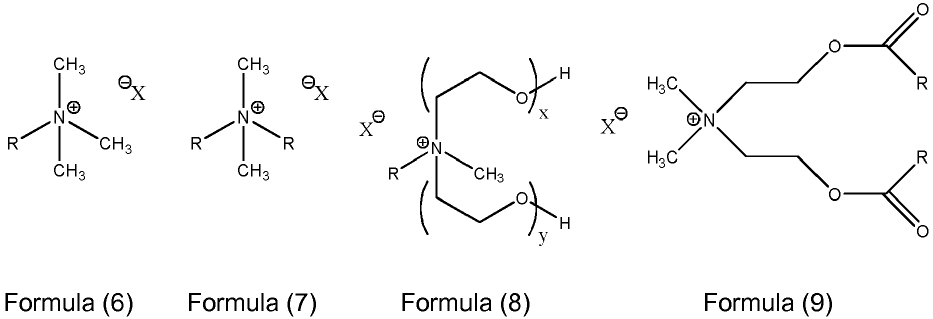 wherein R, x and y have the meanings as defined above for Formulas (2), (3), (4) and (5), and X represents Cl or Br,
by anion exchange reaction with a sodium, potassium, lithium or ammonium salt of (4-chloro-2-methylphenoxy)acetic acid,
wherein the molar ratio of said quaternary ammonium halide to said (4-chloro-2-methylphenoxy)acetic acid salt is in the range from 1:0.95 to 1:1.05, preferably 1:1,
at 273 to 373K, preferably 293K,
in water or in organic solvent selected from the group consisting of methanol, ethanol, propanol, isopropanol, and butanol, or a mixture of water with said organic solvent, to form a compound of Formula (1).

Said exchange reaction can be also carried out in a mixture of two or more organic solvents having molar weight below 100 g/mol.

Thus formed compound of Formula (1) can be subsequently isolated.

If the above reaction is carried out in water, the product of Formula (1) can be isolated from aqueous phase by means of two-phase extraction with an organic solvent selected from the group consisting of chloroform, dichloromethane, toluene, and ethyl acetate, then separation of the organic phase, removal of the organic solvent, and drying the residue, which is the final product.

Alternatively, if the above reaction is carried out in water, the product of Formula (1) can be isolated from the aqueous phase by evaporation of water and addition of an organic solvent selected from the group consisting of acetone, acetonitrile, methanol, ethanol, isopropanol, and ethyl acetate, then filtration of an inorganic by-product from organic solvent, then evaporation of the organic solvent from filtrate, and drying the residue which is the final product.

Furthermore, if the above reaction is carried out in water or in a water-organic solvent mixture, then isolation of the product of Formula (1) may be not necessary. An aqueous or aqueous-organic solution obtained after reaction can be a final product - the ready solution of a herbicidal ionic liquid.

If the above reaction is carried out in an organic solvent, the product of Formula (1) can be isolated by filtration of an inorganic by-product, evaporation of the organic solvent from filtrate, and drying the residue, which is the final product.

Alternatively, salts of the invention of the Formula (1) as defined above, wherein K represents Formula (2), Formula (3), or Formula (4), can be obtained from a quaternary ammonium hydroxide of the Formula (6), Formula (7), or Formula (8), respectively, 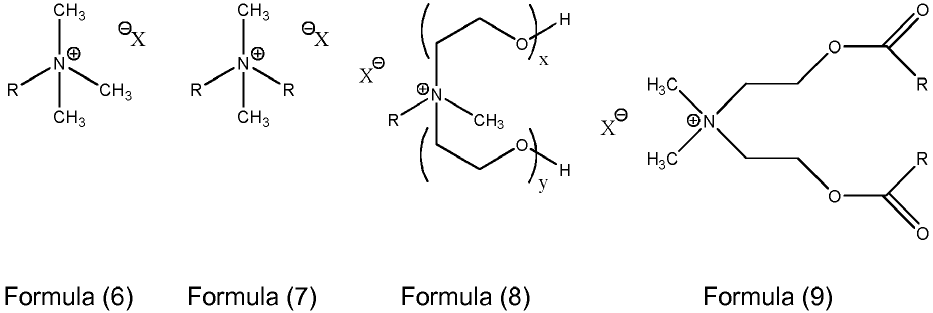 wherein R, x and y have the meanings as defined above for Formulas (2), (3), and (4), and X represents OH, by neutralization reaction with (4-chloro-2-methyl-phenoxy)acetic acid,
wherein the molar ratio of said quaternary ammonium hydroxide to said acid is in the range from 1:0.95 to 1:1.05, preferably 1:1,
at 273 to 373K, preferably 293K,
in water or in organic solvent selected from the group consisting of methanol, ethanol, propanol, isopropanol, and butanol, or the mixture of water with said organic solvent, to form a compound of Formula (1).

Said neutralization reaction can be also carried out in a mixture of two or more organic solvents having molar weight below 100 g/mol.

Thus formed compound of Formula (1) can be subsequently isolated.

If the above reaction is carried out in water, the product of Formula (1) can be isolated from aqueous phase by means of two-phase extraction with an organic solvent selected from the group consisting of chloroform, dichloromethane, toluene, and ethyl acetate, then separation of the organic phase, removal of the organic solvent, and drying the residue, which is the final product.

If the above reaction is carried out in an organic solvent, the product of Formula (1) can be evaporation of the organic solvent from filtrate followed by drying the residue which, is the final product.

Furthermore, if the above reaction is carried out in water or in a water-organic solvent mixture, then isolation of the product of Formula (1) may be not necessary. An aqueous or aqueous-organic solution obtained after reaction can be a final product - ready solution of a herbicidal ionic liquid.

The invention will be illustrated below in details by means of the non-limiting examples.

### Examples

### Example 1

### Preparation of trimethylsoyaammonium (4-chloro-2-methylphenoxy)acetate [TMSA][MCPA]

Trimethylsoyaammonium chloride (41.3 g, 0.12 mol) was dissolved in 100 cm³ of distilled water. Then stoichiometric amount (0.12 mol) of sodium (4-chloro-2-methylphenoxy)acetate dissolved in 50 cm³ of distilled water was added in small portions with constant stirring. Water was evaporated from the reaction mixture and 50 cm³ of acetone were added, and sodium chloride precipitated from the mixture was filtered off (7.0 g, 0.12 mol). Solvent was evaporated from the filtrate, and obtained residue of the product was dried for 24 hours at 323K under reduced pressure. Trimethylsoyaammonium (4-chloro-2-methylphenoxy)acetate was obtained with the yield of 94%.

The structure of the compound was confirmed by proton and carbon nuclear magnetic resonance. ¹H NMR (CDCl₃) δ [ppm] = 0.89 (t, *J* = 6.6 Hz, 3H); 1.27 (m, 20H); 1.68 (s, 2H); 2.05 (m, 4H); 2.21 (s, 3H); 3.34 (s, 9H); 3.45 (m, 2H); 4.39 (s, 2H); 5.33 (m, 2H); 6.89 (d, *J* = 8.9 Hz, 1H); 7.17 (d, *J* = 8.2 Hz, 1H); 7.09 (s, 1H);
¹³C NMR (CDCl₃) δ [ppm] = 170.21; 155.12; 129.44; 129.21; 128.99; 128.69; 126.21; 124.52; 112.32; 65.11; 63.72; 52.21; 33.61; 31.69; 29.76; 29.55; 29.40; 29.17; 29.01; 28.88; 28.72; 25.61; 22.22; 22.06; 15.24; 13.90.

Biphasic titration performed in accordance with the standard PN-EN ISO 2871-1:2000 showed the content of cationically active compound, i.e. [TMSA][MCPA], at 97%.

### Example 2

### Preparation of dimethylditallowammonium (4-chloro-2-methylphenoxy)acetate [DMDTA][MCPA]

Dimethylditallowammonium hydroxide (44.2 g, 0.08 mol) was dissolved in 80 cm³ of methanol. Then stoichiometric amount (0.08 mol) of potassium (4-chloro-2-methylphenoxy)acetate dissolved in 30 cm³ of methanol was added in small portions with constant stirring. Stirring was continued for 10 minutes at 293K, then solvent was evaporated and the reaction product was dried for 24 hours at 323K under reduced pressure. Dimethylditallowammonium (4-chloro-2-methylphenoxy)-acetate was obtained with the yield of 94%.

The structure of the compound was confirmed by proton and carbon nuclear magnetic resonance.
¹H NMR (CDCl₃) δ [ppm] = 0.88 (t, *J* = 6.9 Hz, 6H); 1.27 (m, 40H); 1.72 (s, 4H); 2.09 (m, 8H); 2.18 (s, 3H); 3.29 (s, 6H); 3.45 (m, 4H); 4.39 (s, 2H); 5.40 (m, 4H); 6.80 (d, *J* = 8.5 Hz, 1H); 7.08 (d, *J* = 8.6 Hz, 1H); 7.21 (s, 1H); ¹³C NMR (CDCl₃) δ [ppm] = 170.50; 155.43; 129.99; 129.70; 129.02; 128.79; 126.28; 124.74; 112.22; 65.70; 63.18; 52.34; 33.76; 31.58; 29.97; 29.84; 29.63; 29.51; 29.30; 29.09; 28.77; 28.54; 25.42; 22.77; 22.20; 15.29; 13.72.

Two-phase titration performed in accordance with the standard PN-EN ISO 2871-1:2000 showed the content of cationically active compound, i.e. [DMDTA][MCPA], at 98%.

### Example 3

### Preparation of bis(2-hydroxyethyl)methyloleylammonium (4-chloro-2-methyl-phenoxy)acetate [HMOA][MCPA]

76.9 g of 25% aqueous solution of bis(2-hydroxyethyl)methyloleylammonium hydroxide (0.05 mol) were placed in the flask and 11.9 g (0.05 mol) of potassium (4-chloro-2-methylphenoxy)acetate were added in small portions, with constant stirring. Stirring was continued for 20 minutes at 293K, and then the product was extracted from the mixture with dichloromethane. Organic phase was washed three times with water, and then solvent was evaporated under reduced pressure. The product was dried for 24 hours at 323K under reduced pressure to afford the final product with the yield of 90%.

The structure of the compound was confirmed by proton and carbon nuclear magnetic resonance.
¹H NMR (CDCl₃) δ [ppm]= 0.88 (t, *J* = 6.9 Hz, 3H); 1.27 (m, 22H); 1.72 (s, 2H); 2.00 (m, 4H); 2.18 (s, 3H); 3.33 (s, 3H); 3.55 (m, 2H); 3.69 (m, 4H); 4.09 (m, 4H); 4.55 (s, 2H); 5.36 (m, 2H); 5.51 (s, 2H); 6.84 (d, *J* = 8.8 Hz, 1H); 7.19 (d, *J* = 8.6 Hz, 1H); 7.25 (s, 1H); ¹³C NMR δ [ppm] = 170.26; 154.72; 130.08; 129.90; 129.50; 128.21; 126.34; 124.26; 112.77; 65.32; 63.99; 63.81; 55.63; 50.18; 32.50; 31.76; 29.63; 29.54; 29.40; 29.31; 29.18; 29.12; 29.08; 27.10; 27.07; 26.30; 25.18; 22.54; 16.03; 15.89; 14.03.

Two-phase titration performed in accordance with the standard PN-EN ISO 2871-1:2000 showed the content of cationically active compound, i.e. [HMOA][MCPA], at 97%.

### Example 4

### Preparation of dimethyldi(tallowoyloxyethyl)ammonium (4-chloro-2-methylphenoxy)-acetate [DMDTOA][MCPA]

82.0 g of 50% methanol solution of dimethyldi(tallowoyloxyethyl)ammonium chloride (0.06 mole) were placed in the flask and stoichiometric amount (0.06 mol) of sodium (4-chloro-2-methylphenoxy)acetate dissolved in 30 cm³ of methanol was added in small portions with constant stirring. Stirring was continued for 15 minutes at ambient temperature, then 3.5 g (0.06 mole) of the formed by-product sodium chloride were filtered off, the solvent was evaporated from the filtrate, and the reaction product was dried for 24 hours at 323K under reduced pressure. The yield of the reaction was 98%.

The structure of the compound was confirmed by proton and carbon nuclear magnetic resonance.
¹H NMR (CDCl₃) δ [ppm] = 0.88 (t, *J* = 6.6 Hz, 6H); 1.26 (m, 56H); 1.59 (m, 4H); 2.01 (m, 4H); 2.21 (s, 3H); 2.31 (t, *J* = 7.7 Hz, 4H); 3.28 (s, 6H); 3.90 (m, 4H); 4.40 (s, 2H); 4.42 (m, 4H); 5.34 (m, 2H); 6.71 (d, *J* = 8.7 Hz, 1H); 7.02 (d, *J* = 8.6 Hz, 1H); 7.05 (s, 1H); ¹³C NMR (CDCl₃) δ [ppm] = 172.97; 172.51; 172.49; 155.97; 129.91; 129.87; 129.46; 128.24; 125.98; 124.15; 112.66; 68.28; 63.02; 57.38; 51.60; 33.77; 33.75; 31.76; 31.73; 29.59; 29.54; 29.50; 29.47; 29.36; 29.32; 29.20; 29.15; 29.12; 29.04; 28.97; 28.95; 28.94; 27.05; 24.50; 22.52; 16.19; 13.96.

Two-phase titration performed in accordance with the standard PN-EN ISO 2871-1:2000 showed the content of cationically active compound, i.e. [DMDTOA][MCPA], at 98%.

### Example 5

### Preparation of bis(2-hydroxyethyl)methyloleylammonium (4-chloro-2-methyl-phenoxy)acetate [HMOA][MCPA]

192.3 g of 10% aqueous solution of bis(2-hydroxyethyl)methyloleylammonium hydroxide (0.05 mole) were placed in the flask and 11.9 g (0.05 mole) of potassium (4-chloro-2-methylphenoxy)acetate were added in small portions with constant stirring. Stirring was continued for 10 minutes at 293K, to obtain ready solution of herbicidal ionic liquid.

Two-phase titration of the solution performed in accordance with the standard PN-EN ISO 2871-1:2000 showed the content of cationically active compound, i.e. [HMOA][MCPA] at 98% (corresponds to concentration of herbicidal ionic liquid in the solution of 9.8%).

### Example 6

### Preparation of dimethyldi(tallowoyloxyethyl)ammonium (4-chloro-2-methylphenoxy)-acetate [DMDTOA][MCPA]

82.0 g (0.06 mole) of 50% methanolic solution of dimethyldi(tallowoyloxyethyl)-ammonium chloride were placed in the flask and stoichiometric amount (0.06 mole) of sodium (4-chloro-2-methylphenoxy)acetate dissolved in 30 cm³ of water was added in small portions with constant stirring. Stirring was continued for 15 minutes at 293K, to obtain ready solution of herbicidal ionic liquid.

Two-phase titration performed in accordance with the standard PN-EN ISO 2871-1:2000 showed the content of cationically active compound, i.e. [DMDTOA][MCPA] at 99% (corresponds to concentration of herbicidal ionic liquid in the solution of 9.9%).

### Example 7

### Tests of novel herbicidal ionic liquids of the invention

Biological activity of novel ionic liquids was confirmed in field experiments performed under natural weed-growth conditions.

Tests were performed on the plots of the size 2 m x 5 m. Solutions of tested ionic liquids were prepared by dissolving in water a measured amount of the compound corresponding to adopted dosage of MCPA (calculated as the acid) per 1 ha.

The following ionic liquids of the invention were tested:
- [TMSA][MCPA] - trimethylsoyaammonium (4-chloro-2-methylphenoxy)acetate,
- [DMDTA][MCPA] - dimethylditallowammonium (4-chloro-2-methylphenoxy)acetate,
- [HMOA][MCPA] - bis(2-hydroxyethyl)methyloleylammonium (4-chloro-2-methylphenoxy)acetate,
- [DMDTOA][MCPA] - dimethyldi(tallowoyloxyethyl)ammonium (4-chloro-2-methylphenoxy)acetate.

Comparative herbicide (Herbicide X) was commercial formulation containing 300 g of MCPA in the form of sodium-potassium salt form per 1 dm³. This formulation was dissolved in water in accordance with manufacturer's recommendations in the amount corresponding to 400 g MCPA per 1 ha (6.65 cm³ of the formulation per 1 dm³ of water). Comparative objects were plots without use of a herbicide.

All treatments were applied using backpack sprayer equipped with flat spray nozzlesTee Jet 110 03 XR, under constant pressure of 0.2 MPa and application rate 200 dm³ per 1 ha. During treatment weeds were at the following growth stage: goosefoot *(Chenopodium album*) - from 4 to 10 leafs, field penny-cress (*Thlaspi arvense*) *-* blossom phase, *Matricaria inodora -* fully developed rosette, volunteer rapeseed (*Brassica napus*) *-* 4-6 leafs.

Typical methodology adopted for this type of test was used to determine efficacy of combatting weeds, i.e. the degree of eradication of a given weed species on each plot treated with tested compound in comparison with corresponding control plot was assessed. The efficacy of tested compounds was presented in percents, where 100% corresponds to total weeds destruction (weeds completely dried), and 0% corresponds to lack of herbicidal activity. The results of the efficacy tests were presented as a mean of assessment of weeds eradication from four replicates.

The results of activity of representative ionic liquids are presented in Tables 1-3.

**Table 1. Weed control in winter wheat**

| Object | Dose of MCPA g/ha | *Brassica napus* | *Thlaspi arvense* | *Matricaria inodora* |
|---|---|---|---|---|
| | | ------- % of weed control ------ | | |
| [TMSA][MCPA] | 400 | 100 | 100 | 70 |
| [DMDTA][MCPA] | 400 | 100 | 100 | 62 |
| Herbicide X | 900* | 100 | 100 | 56 |

| | | | | |
|---|---|---|---|---|
| *dose recommended for use in winter wheat | | | | |

**Table 2. Chenopodium album control in spring barley**

| Object | Dose of MCPA g/ha | % of weed control |
|---|---|---|
| [HMOA][MCPA] | 400 | 60 |
| [HMOA][MCPA] | 600 | 80 |
| Herbicyd X | 400 | 40 |
| Herbicide X | 600 | 70 |

**Table 3. Chenopodium album control in spring barley**

| Object | Dose of MCPA g/ha | % of weed control |
|---|---|---|
| [DMDTOA][MCPA] | 400 | 90 |
| Herbicyd X | 400 | 40 |

Field experiments showed that ionic liquids - salts containing MCPA anion of the invention were biologically active in similar or better degree comparing to currently recommended herbicide containing MCPA as sodium-potassium salts. Novel herbicidal ionic liquids did not cause destruction of winter wheat and spring barley plants.

### Example 8

### Comparison of ionic liquids of the invention with prior art compounds

Field experiments were performed on the plots of dimensions 1.5 m x 11 m with spring barley. Solutions of tested ionic liquids were prepared by dissolving in water a measured amount of the compound corresponding to adopted dosage of MCPA (calculated as the acid) per 1 ha.

The following representative compounds of the invention were tested:
- [DMDTOA][MCPA] - dimethyldi(tallowoyloxyethyl)ammonium (4-chloro-2-methylphenoxy)acetate, and
- [TMSA][MCPA] - trimethylsoyaammonium (4-chloro-2-methylphenoxy)acetate.

As a comparison, the following specific compounds disclosed in WO2008/140338 and in J. Pernak et al., Tetrahedron 67(2011), 4838-4844, were tested:
- [DMDHTA][MCPA] - dimetylodi(hydrogenated tallow)ammonium (4-chloro-2-methylphenoxy)acetate, and
- [DDA][MCPA] - didecyldimethylammonium (4-chloro-2-methylphenoxy)acetate. Efficacy of *Chenopodium album* control in spring barley was assessed after treatments performed according to the procedure described in Example 7. Results are presented below in Table 4.

**Table 4. Chenopodium album control in spring barley**

| Object | R substitent | Dose of MCPA g/ha | % of weed control |
|---|---|---|---|
| [DMDTOA][MCPA] | mixture of unsaturated and saturated | 400 | 98 |
| [TMSA][MCPA] | mixture of unsaturated and saturated | 400 | 100 |
| [DMDHTA][MCPA] | saturated | 400 | 94 |
| [DDA][MCPA] | saturated | 400 | 94 |

Comparison of efficacy of herbicidal ionic liquids of the invention with prior art ionic liquids in *Chenopodium album* control in spring barley demonstrated that herbicidal ionic liquids of the invention containing mixture of unsaturated and saturated long-chain alkyl substituents were from 4 to 6% more effective than prior art ionic liquids containing solely saturated long-chain alkyl substituents.

## Claims

1. A salt represented by the following Formula (1) 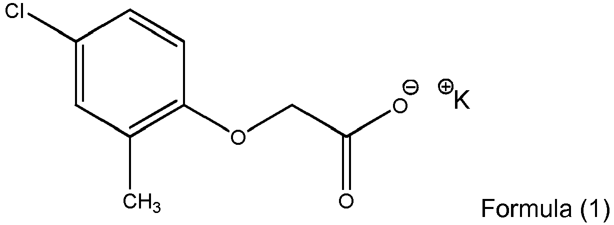 wherein K represents a quaternary ammonium cation of the formula selected from the group consisting of Formula (2), Formula (3), Formula (4), and Formula (5), 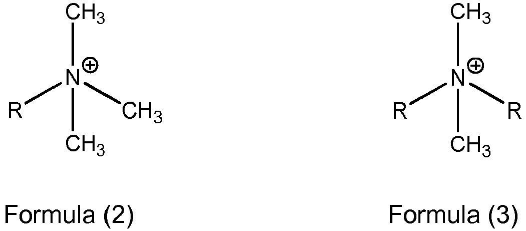 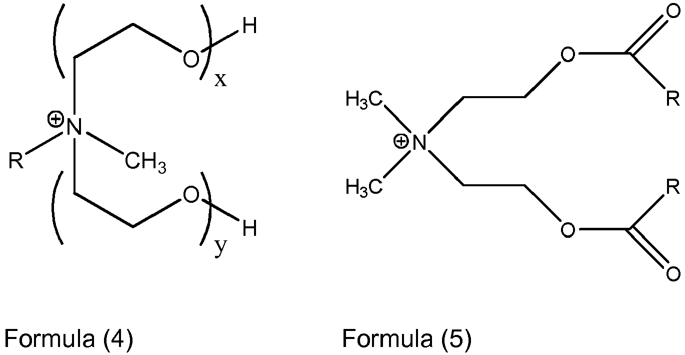 wherein R represents an alkyl selected from the group consisting of tallow, soya and oleyl alkyl, and
x and y in Formula (4) are integers from the range 1 to 15, inclusive;
and wherein
tallow alkyl relates to a mixture of saturated and unsaturated alkyl groups composed of alkyl groups with 12 carbon atoms, 14 carbon atoms, 16 carbon atoms, and 18 carbon atoms, wherein the percentage of groups with double bonds is 40 to 55%, the percentage of alkyl groups with 16 carbon atoms is 25 to 35%, and the percentage of alkyl groups with 18 carbon atoms is 60 to 70%;
soya alkyl relates to a mixture of saturated and unsaturated alkyl groups composed of alkyl groups with 14 carbon atoms, 16 carbon atoms, and 18 carbon atoms, wherein the percentage of groups with double bonds is 55 to 70%; and
oleyl alkyl relates to a mixture of saturated and unsaturated alkyl groups composed of alkyl groups having 12 carbon atoms, 14 carbon atoms, 16 carbon atoms, and 18 carbon atoms, wherein the percentage of groups with double bonds is 75 to 82%, and the percentage of alkyl groups with 18 carbon atoms is 75 to 85%.

2. The salt according to claim 1, wherein R represents tallow alkyl.

3. The salt according to claim 1, wherein R represents soya alkyl.

4. The salt according to claim 1, wherein R represents oleyl alkyl.

5. The salt according to any one of claims 1 to 4, wherein K in Formula (1) is selected from the group consisting of Formula (2) and Formula (3).

6. The salt according to any one of claims 1 to 4, wherein K in Formula (1) is selected from the group consisting of Formula (4) and Formula (5).

7. The salt according to any one of claims 1 to 5, wherein K in Formula (1) is represented by Formula (2).

8. The salt according to any one of claims 1 to 5, wherein K in Formula (1) is represented by Formula (3).

9. The salt according to any one of claims 1 to 4 and 6, wherein K in Formula (1) is represented by Formula (4).

10. The salt according to any one of claims 1 to 4 and 6, wherein K in Formula (1) is represented by Formula (5).

11. The salt according to claim 1, selected from the following group:
- trimethylsoyaammonium (4-chloro-2-methylphenoxy)acetate,
- dimethylditallowammonium (4-chloro-2-methylphenoxy)acetate
- bis(2-hydroxyethyl)methyloleylammonium (4-chloro-2-methylphenoxy)acetate, and
- dimethyldi(tallowoyloxyethyl)ammonium (4-chloro-2-methylphenoxy)acetate.

12. A herbicidal composition, which comprises a herbicide and a carrier, **characterized in that** the herbicide is the salt as defined in any one of claims 1 to 11.

13. A method of combating undesirable plant vegetation, which comprises applying a herbicidally active amount of the salt as defined in any one of the claims 1 to 11 or the composition as defined in claim 12 to said undesirable plant vegetation or locus thereof.

## Patentansprüche

1. Ein Salz, dargestellt durch die folgende Formel (1) 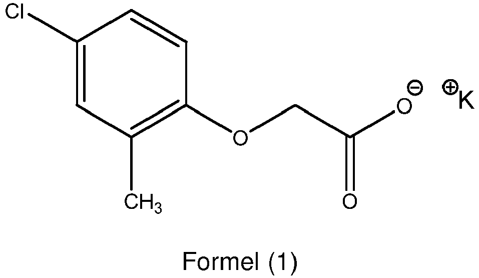 worin K ein quaternäres Ammoniumkation der Formel ausgewählt aus der Gruppe bestehend aus Formel (2), Formel (3), Formel (4) und Formel (5), darstellt 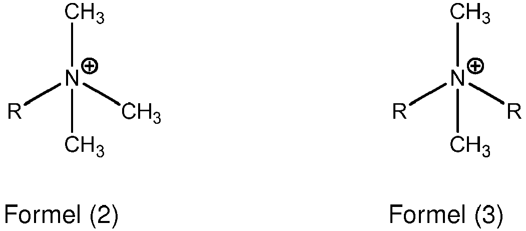 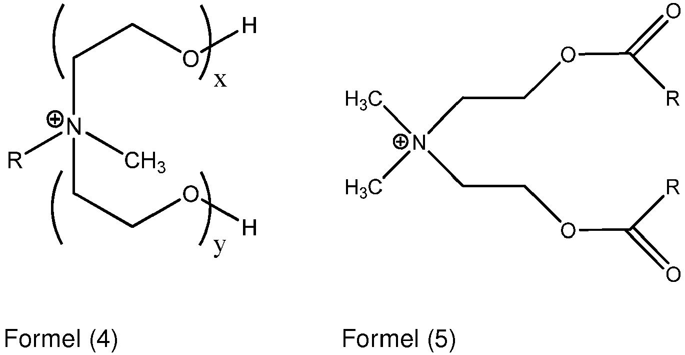 worin R für ein Alkyl steht, ausgewählt aus der Gruppe bestehend aus Talg-, Soja- und Oleylalkyl, und x und y in Formel (4) sind ganze Zahlen im Bereich von 1 bis 15, einschließlich,
worin
Talgalkyl bezieht sich auf ein Gemisch von gesättigten und ungesättigten Alkylgruppen bestehend aus Alkylgruppen mit 12 Kohlenstoffatomen, 14 Kohlenstoffatomen, 16 Kohlenstoffatomen und 18 Kohlenstoffatomen, wobei der Anteil der Gruppen mit Doppelbindungen 40 bis 55% beträgt, der Anteil der Alkylgruppen mit 16 Kohlenstoffatomen 25 bis 35% beträgt und der Anteil der Alkylgruppen mit 18 Kohlenstoffatomen 60 bis 70% beträgt,
Soja-Alkyl bezieht sich auf ein Gemisch von gesättigten und ungesättigten Alkylgruppen bestehend aus Alkylgruppen mit 14 Kohlenstoffatomen, 16 Kohlenstoffatomen und 18 Kohlenstoffatomen, wobei der Anteil der Gruppen mit Doppelbindungen 55 bis 70 beträgt %, und
Oleylalkyl bezieht sich auf ein Gemisch von gesättigten und ungesättigten Alkylgruppen bestehend aus Alkylgruppen mit 12 Kohlenstoffatomen, 14 Kohlenstoffatomen, 16 Kohlenstoffatomen und 18 Kohlenstoffatomen, wobei der Anteil der Gruppen mit Doppelbindungen 75 bis 82% beträgt und der Anteil der Alkylgruppen mit 18 Kohlenstoffatomen 75 bis 85% beträgt.

2. Salz nach Anspruch 1, worin R für Talgalkyl steht.

3. Salz nach Anspruch 1, worin R für Sojaalkyl steht.

4. Salz nach Anspruch 1, worin R für Oleylalkyl steht.

5. Salz nach einem der Ansprüche 1 bis 4, worin K in Formel (1) ausgewählt ist aus der Gruppe bestehend aus Formel (2) und Formel (3).

6. Salz nach einem der Ansprüche 1 bis 4, worin K in Formel (1) ausgewählt ist aus der Gruppe bestehend aus Formel (4) und Formel (5).

7. Salz nach einem der Ansprüche 1 bis 5, worin K in Formel (1) durch Formel (2) dargestellt ist.

8. Salz nach einem der Ansprüche 1 bis 5, worin K in Formel (1) durch die Formel (3) dargestellt ist.

9. Salz nach einem der Ansprüche 1 bis 4 und 6, worin K in Formel (1) durch Formel (4) dargestellt ist.

10. Salz nach einem der Ansprüche 1 bis 4 und 6, worin K in Formel (1) durch Formel (5) dargestellt ist.

11. Salz nach Anspruch 1, ausgewählt aus der folgenden Gruppe:
- Trimethylsojaammonium (4-chlor-2-methylphenoxy)acetat,
- Dimethylditalgammonium (4-chlor-2-methylphenoxy)acetat,
- bis (2-Hydroxyethyl)methyloleylammonium (4-chlor-2-methylphenoxy)acetat, und
- Dimethyldi(talgoyloxyethyl) ammonium (4-chlor-2-methylphenoxy)acetat.

12. Eine herbizide Zusammensetzung, die ein Herbizid und einen Träger umfasst, **dadurch gekennzeichnet, dass** das Herbizid das Salz nach einem der Ansprüche 1 bis 11 ist.

13. Verfahren zur Bekämpfung von unerwünschter Pflanzenvegetation, welches das Auftragen einer herbizid wirksamen Menge des in einem der Ansprüche 1 bis 11 definierten Salzes oder der in Anspruch 12 definierten Zusammensetzung auf die unerwünschte Pflanzenvegetation oder deren Lage umfasst.

## Revendications

1. Un sel représenté par la Formule (1) suivante 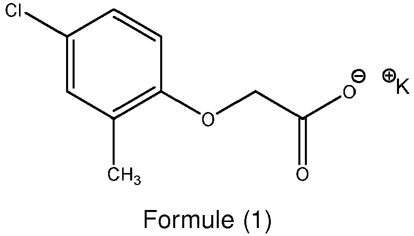 dans lequel K représente un cation d'ammonium quaternaire de formule choisie dans le groupe constitué par la formule (2), la formule (3), la Formule (4) et la Formule (5), 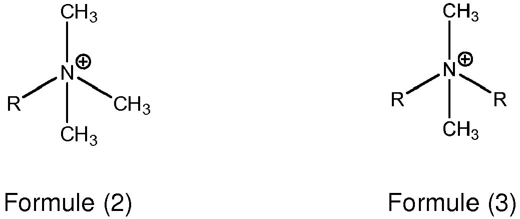 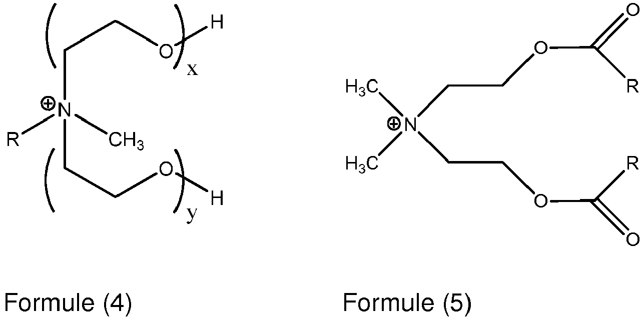 dans lequels R représente un alkyle choisi dans le groupe consistant en alkyle de suif, de soja et d'oléyl, et x et y dans la formule (4) sont des nombres entiers allant de 1 à 15, inclus,
et dans lequels
alkyle de suif concerne un mélange de groupes alkyle saturés et insaturés composés de groupes alkyle avec 12 atomes de carbone, 14 atomes de carbone, 16 atomes de carbone et 18 atomes de carbone, dans lequel le pourcentage de groupes à double liaison est de 40 à 55%, le pourcentage de groupes alkyle avec 16 atomes de carbone est de 25 à 35% et le pourcentage de groupes alkyle avec 18 atomes de carbone est de 60 à 70%,
alkyle de soja concerne un mélange de groupes alkyle saturés et insaturés composés de groupes alkyle avec 14 atomes de carbone, 16 atomes de carbone et 18 atomes de carbone, dans lequel le pourcentage de groupes à double liaison est de 55 à 70 %, et
alkyle d'oléyle concerne un mélange de groupes alkyle saturés et insaturés composés de groupes alkyle ayant 12 atomes de carbone, 14 atomes de carbone, 16 atomes de carbone et 18 atomes de carbone, dans lequel le pourcentage de groupes à double liaison est de 75 à 82%, et le pourcentage de groupes alkyle avec 18 atomes de carbone est de 75 à 85%.

2. Le sel selon la revendication 1, dans lequel R représente un alkyle de suif.

3. Le sel selon la revendication 1, dans lequel R représente un alkyle de soja.

4. Le sel selon la revendication 1, dans lequel R représente un alkyle d'oléyle.

5. Le sel selon l'une quelconque des revendications 1 à 4, dans lequel K dans la formule (1) est choisi dans le groupe constitué par la Formule (2) et la Formule (3).

6. Le sel selon l'une quelconque des revendications 1 à 4, dans lequel K dans la Formule (1) est choisi dans le groupe constitué par la Formule (4) et la Formule (5).

7. Le sel selon l'une quelconque des revendications 1 à 5, dans lequel K dans la formule (1) est représenté par la Formule (2).

8. Le sel selon l'une quelconque des revendications 1 à 5, dans lequel K dans la Formule (1) est représenté par la Formule (3).

9. Sel selon l'une quelconque des revendications 1 à 4 et 6, dans lequel K dans la Formule (1) est représenté par la Formule (4).

10. Le sel selon l'une quelconque des revendications 1 à 4 et 6, dans lequel K dans la Formule (1) est représenté par la Formule (5).

11. Sel selon la revendication 1, choisi dans le groupe suivant:
- (4-chloro-2-méthylphénoxy)acétate de triméthylsoyaammonium,
- (4-chloro-2-méthylphénoxy)acetate de diméthyldi(suif)ammonium
- (4-chloro -2-méthylphénoxy)acétate de bis(2-hydroxyéthyl)méthyloléylammonium, et
- (4-chloro-2-méthylphénoxy)acétate de diméthyldi((suifoyloxyéthyl)ammonium.

12. Composition herbicide, qui comprend un herbicide et un support, **caractérisé en ce que** l'herbicide est le sel tel que défini dans l'une quelconque des revendications 1 à 11.

13. Procédé de lutte contre la végétation végétale indésirable, qui comprend l'application d'une quantité herbicide efficace du sel tel que défini dans l'une quelconque des revendications 1 à 11 ou la composition telle que définie dans la revendication 12 à ladite végétation végétale indésirable ou au lieu de celle-ci.
